## Europäisches Patentamt
## European Patent Office
## Office européen des brevets

(19)

(11) Veröffentlichungsnummer: **0 052 074**
**B1**

(12) **EUROPÄISCHE PATENTSCHRIFT**

(45) Veröffentlichungstag der Patentschrift:
04.02.87

(51) Int. Cl.⁴: **A 61 K 9/00**, A 61 K 9/70

(21) Anmeldenummer: **81810442.4**

(22) Anmeldetag: **06.11.81**

(54) Verwendung von anticholinergen Substanzen.

(30) Priorität: **12.11.80 CH 8391/80**

(43) Veröffentlichungstag der Anmeldung:
**19.05.82 Patentblatt 82/20**

(45) Bekanntmachung des Hinweises auf die Patenterteilung:
**04.02.87 Patentblatt 87/6**

(84) Benannte Vertragsstaaten:
**CH DE FR GB IT LI LU NL SE**

(56) Entgegenhaltungen:
**US-A-4 262 003**

**REMINGTON'S PHARMACEUTICAL SCIENCES, ed. 14, 1970, Mack Publ. Comp., EASTON, PENNSYLVANIA (US), Seiten 914-927**
**CHEMICAL ABSTRACTS, Band 69, no. 23, 2. Dezember 1968, Seite 8880, Zusammenfassung No. 94981v, COLUMBUS OHIO (US)J. MOLLER et al. "Comparative studies on intramuscular and oral effective doses of some anticholinergic drugs"**
**CHEMICAL ABSTRACTS, Band 74, no. 17, 26 April 1971, Seite 301, Zusammenfassung no. 86265c, COLUMBUS OHIO (US), W.J. TAYLOR et al.: "Comparative evaluation of intramuscular atropine, dicyclomine, and glycopyrrolate using healthy medical students as volunteer subjects"**
**CHEMICAL ABSTRACTS, Band 69, no. 23, 2. Dezember 1968, Seite 8853, Zusammenfassung no.**

(73) Patentinhaber: **ALZA CORPORATION, 950 Page Mill Road, Palo Alto, California 94304 (US)**

(72) Erfinder: **Bieck, Peter, Prof. Dr., Spitzbergweg 3, D-7407 Rottenburg- Wurmlingen (DE)**
Erfinder: **Fara, John William, Dr., 12138 Scully Avenue, Saratoga California 95070 (US)**
Erfinder: **Shaw, Jane Elizabeth, Dr., 1 Larch Avenue, Atherton California 94025 (US)**
Erfinder: **Urquhart, John, Dr., 975 Hamilton Avenue, Palo Alto California 94301 (US)**

(74) Vertreter: **Meyer, Hans Rudolf, Patentabteilung der CIBA- GEIGY AG Postfach, CH- 4002 Basel (CH)**

(56) Entgegenhaltungen: (Fortsetzung)
**94716n, COLUMBUS OHIO (US), R. BAUER et al.: "Difference of action of atropine, scopolamine, and some of their quaternary derivatives after subcutaneous and eternal administration, with special consideration to scopolamine butybromide"**
**CHEMISCHES ZENTRALBLATT, Nr. 26, 28. Juni 1967, Zusammenfassung 1866, J. MANNHEIM: "Injizierbare pharmazeutische Gemische"**
**"The Pharmacological Basis of Therapeutics", L.S. Goodman & A. Gilman, McNilton Publ.Co.Inc., New York, US, 6th Ed., 1980, pp. 120-136**

EP 0 052 074 B1

**0 052 074**

**Beschreibung**

Die vorliegende Erfindung betrifft die Verwendung von Scopolamin als anticholinerge Substanz zur Herstellung eines transdermal wirkenden Arzneimittels, welches Scopolamin kontinuierlich abgibt und zur Hemmung der Magensäuresekretion verabreicht wird. Das transdermal wirkende Arzneimittel ist für die Behandlung von Hyperacidität und Ulcuskrankheiten besonders geeignet.

Es ist bekannt, dass man zur Behandlung von Hyperacidität und Ulcuskrankheiten anticholinergisch wirksame Substanzen oral verabreichen kann. Nach der neuesten Auflage des Lehrbuches "The Pharmacological Basis of Therapeutics" von L.S. Goodman and A. Gilman (Mac Millan Publ. Co., Inc. New York, 1980, 6th ed.) beeinflussen muscarinähnliche cholinerg-blockierende Substanzen vom Atropin- oder auch Scopolamintypus die Magensäuresekretion. Es wird jedoch darauf verwiesen, dass nur verhältnismässig hohe Dosen wirksam sind. Nach dieser heute gültigen Auffassung sind tägliche Dosen, die an die subtoxische Grenze reichen, zur Hemmung der Magensekretion erforderlich. Beispielsweise sind bei Anwendung von Scopolamin Dosen von 1 mg und mehr notwendig, um die gewünschte Magensäuresekretion am Menschen zu hemmen. Solche hohen Dosen verursachen jedoch fast immer beträchtliche Nebenwirkungen, wie z.B. Mundtrockenheit, Sehstörungen, Photophobie, Pulsbeschleunigung und Harnentleerungsstörungen. K.J. Ivey, Gastroenterology 68, 154-166 (1975) kommt nach Durchsicht von ca. 400 Arbeiten über die Anwendung von Anticholinergica bei der Ulcuskrankheit zur gleichen Schlussfolgerung. Die auftretenden Nebenwirkungen sind so unerträglich, dass viele Patienten die Behandlung nicht längere Zeit tolerieren.

Nach Untersuchungen von M. Feldman et al. (N. Engl. J. Med. 297, 1427-1430 (1977) wird die bisher allgemein vertretene Meinung, dass sich eine Hemmung der Magensekretion nur durch sehr hohe Dosen eines verabreichten Parasympatholyticums erreichen lässt, allerdings in Frage gestellt. Die oben erwähnten Autoren stellten fest, dass eine niedrige Dosis (15 mg) Propanthelin, die durch Nahrungsaufnahme induzierte Magensäuresekretion bei Patienten mit Ulcus duodeni in gleichem Masse hemmt, wie eine subtoxische Dosis von 48 mg. Ferner wurde festgestellt, dass diese niedrigere Dosis Propanthelin in Kombination mit einem $H_2$-Rezeptor-Antagonisten, wie z.B. Cimetidin, die Säuresekretion stärker hemmte als beide Arzneistoffe alleine. Dieses Konzept fand wegen der nicht sicheren Wirksamkeit selbst bei relativ hohen Dosen keine verbreitete Anwendung. Aus der US-A 3 996 934 ist lediglich die eventuelle transdermale Anwendung von Anticholinergica ohne besondere therapeutische Verwendungsangaben ersichtlich. Die Verwendung von Anticholinergica zur Herstellung eines transdermalen Arzneimittels zur Hemmung der Magensäuresekretion wird nicht erwähnt. Aus Chemical Abstracts 69:94716h, (1968) R. Bauer et al. ist bekannt, dass die anticholinergen Substanzen die Magensäuresekretion hemmen, wenn sie auf verschiedenem Wege verabreicht werden. Man findet jedoch keinen spezifischen Hinweis auf die Verwendung von Anticholinergica zur Herstellung eines transdermal wirkenden Arzneimittels zur Hemmung der Magensäuresekretion. Es wird lediglich von Untersuchungen an Mäusen und Ratten berichtet, die zeigen, dass es prinzipiell möglich ist, die Magensäuresekretion zu hemmen. Es wird dabei festgestellt, dass bei parentaler Verabreichung eine vielfach stärkere Wirkung als bei der Gabe in das Duodenum erzielt wird. Aus diesen Untersuchungen ist kein Widerspruch zur bekannten Lehrmeinung zu entnehmen, dass nur grosse Dosen von Anticholinergica den gewünschten Effekt herbeiführen.

Aus Chemical Abstracts 74, 86265c (1971) ist zu entnehmen, dass Atropin in der verwendeten hohen parenteralen Dosis (1,5 mg i.p.) die Säuresekretion und den Speichelfluss hemmt. Gleichzeitig wird aber auch die Pulsfrequenz gesteigert. Dieser ungewollte und eher als schädlich zu bezeichnende Nebeneffekt tritt bei der Verwendung von Scopolamin in einem Arzneimittel in transdermaler Form nicht auf, zumal Scopolamin nur in geringer Dosis verwendet wird.

Aus Remington's Pharmaceutical Sciences, 1970 14. Aufl.) ist bekannt, dass eine Anzahl von anticholinergen Substanzen die Magensäuresekretion hemmen, wenn sie subkutan verabreicht werden, doch wird der therapeutische Nutzen der anticholinergen Substanzen als solche bei Ulcuskrankheiten wegen der nicht zuverlässigen Wirkung und der schlechten Tolerierbarkeit bei oraler Applikation generell in Frage gestellt (z.B. Valethanat-Bromid, Anisotropin, Methylbromid, Dibutolin, Oxyphenonium-Bromid und Poldin). Parenteral jedoch können diese Substanzen appliziert werden, wobei die kontinuierliche Abgabe des Wirkstoffes in kontrollierter Dosis, wie in der transdermalen Form vorgesehen, entfällt.

Es wurde nun überraschenderweise festgestellt, dass bei Verwendung von Scopolamin als anticholinerge Substanz in Form eines transdermal zu verabreichenden Systems, welche das Scopolamin kontinuierlich abgibt, die Magensäuresekretion wirksam gehemmt wird, wobei die unerwünschten Nebenwirkungen zurück oder gänzlich verdrängt werden.

Die Erfindung betrifft vor allem die Verwendung von Scopolamin als Anticholinergicum zur Herstellung eines transdermal wirkenden Arzneimittels zur Hemmung der Magensäuresekretion, welches das Scopolamin in einer Menge von 0,3-15 µg/h kontinuierlich abgibt.

Vor allem betrifft die Erfindung die Verwendung von Scopolaminbase als Anticholinergicum zur Herstellung eines transdermal wirkenden Arzneimittels, welches das Scopolamin kontinuierlich in einer Menge von 0,3 bis 15 µg/h abgibt.

Insbesondere betrifft die Erfindung die Verwendung von Scopolaminbase als Anticholinergicum zur Herstellung eines transdermal wirkenden Arzneimittels, welches das Scopolamin in einer Anfangsstossmenge von 10 bis 200 µg/cm² Haut und anschliessend kontinuierlich in einer Menge von 0,3 bis 15 µg/h abgibt, zur Hemmung der Magensäuresekretion.

Das transdermal wirkende Arzneimittel, welches Scopolamin kontinuierlich in einer Menge von 0,3 bis 15

2

µg/h abgibt kann auch als Ergänzung bei der Verwendung von Antacida und/oder $H_2$-Rezeptor-Antagonisten zur Hemmung der Magensäuresekretion verabreicht werden.

Die Antacida und/oder $H_2$-Rezeptor-Antagonisten können oral oder auch parenteral verabreicht werden.

Als $H_2$-Rezeptor-Antagonisten können Substanzen verwendet werden, die eine stark hemmende Wirkung auf die histamininduzierte Magensaftsekretion aufweisen. Als $H_2$-Rezeptor-Antagonisten kommen beispielsweise Ranitidin, Metiamid, Burimamid, Tiotidin und insbesondere Cimetidin und deren therapeutisch wirksamen Salze in Frage.

Als Antacida können beispielsweise die von B. und H.H. Helwig (Moderne Arzneimittel, 5. Auflage, S. 656-665, 1980) beschriebenen Präparate verabreicht werden. Insbesondere können Metallsalze, wie z.B. Alkali-, Erdalkali- und auch Metallsalze aus der III. Gruppe des periodischen Systems verwendet werden. Von besonderer Bedeutung sind z.B. die Natrium-, Calcium- oder Magnesiumcarbonate, bzw. -bicarbonate, oder auch Aluminiumsilikate. Es können auch andere durch Hydrolyse einen Aluminiumhydroxidfilm ergebende komplexe Doppelsilikate, beispielsweise Natriumaluminiumsilikat, verwendet werden.

Als weitere säurehemmende Substanzen können Pepstatin und auch cytoprotektive Substanzen, wie z.B. verschiedene Prostaglandine, beispielsweise $PGE_2$ oder auch 16,16-Dimethyl-$PGE_2$, verwendet werden.

Die vorher erwähnten transdermalen Systeme ermöglichen eine kontinuierliche Wirkstoffabgabe mit definierter Geschwindigkeit und Dauer.

Die transdermalen therapeutischen Systeme kontrollieren die Wirkstoffabgabe an der Oberfläche intakter Haut. Die Geschwindigkeit der Abgabe liegt dabei wesentlich unterhalb der maximalen Aufnahmefähigkeit der Haut. Der Wirkstoff diffundiert durch die Epidermis und gelangt über die Kapillaren in den Kreislauf.

Als transdermale therapeutische Systeme können beispielsweise die in den U.S. Patenten Nr. 3,598,122, 3,598,123, 3,597,494 und 4,060,084 beschriebenen vorzugsweise das in der DOS Nr. 26 04 718 bzw. in den U.S. Patenten 4,031,894 und 4,262,003 beschriebene transdermale therapeutische System zur Anwendung kommen, wobei die erfindungsgemässe Anwendung nicht auf diese in den Patenten oder in der Offenlegungsschrift dargelegten transdermalen therapeutischen Systeme beschränkt ist. Das transdermale therapeutische System gemäss DOS 26 04 718 ist ein therapeutisches Präparat in Form einer pflasterartigen Auflage, die Scopolaminbase transdermal zunächst in einer Anfangsstossmenge von 10 bis 200 µg/cm$^2$ Haut abgibt und dadurch die Konzentration an Wirkstoff im Plasma schnell auf einen Wert bringt, bei dem es ohne die bereits oben erwähnten unangenehmen Nebenwirkungen die Magensäuresekretion hemmt und anschliessend in einer Menge von 0,3 bis 15 µg/h freisetzt, so dass der Wirkstoffgehalt im Plasma annähernd konstant aufrechterhalten bleibt.

In den meisten Fällen liegt der Anfangsstoss im Bereich von 50-150 µg Scopolamin pro cm$^2$ behandelter Haut.

Die Konzentration von Scopolamin in dem Plasma kann mit der Konzentration des freien Scopolamins im Urin in Zusammenhang gebracht werden. Es zeigt sich, dass eine mittlere Ausscheidungsgeschwindigkeit im Urin von ungefähr 0,5 µg freies Scopolamin pro Stunde im allgemeinen einem therapeutischen Gehalt im Plasma entspricht. Es hat sich jedoch auch gezeigt, dass diese Geschwindigkeit einer ungefähr ± 5-fachen biologischen Variation unterliegt. Daher liegt die Geschwindigkeit im Bereich von ungefähr 0,1 bis ungefähr 2,5 µg/h je nach dem einzelnen Individuum.

Der Grund für die anschliessende, im wesentlichen konstante Abgabegeschwindigkeit im dem Dosierungsprogramm liegt darin, soweit nötig, den Wirkstoffstoss zu unterstützen, indem ausreichend Scopolamin verabreicht wird, um die oben angegebene therapeutische Menge im Plasma zu erzielen und diesen Gehalt solange wie möglich aufrechtzuerhalten. Daraus folgt, dass die Verabreichung mit konstanter Geschwindigkeit solange anhalten soll, wie die Therapie es erfordert. In diesem Zusammenhang ergibt eine Gesamtmenge (einschliesslich dem Stoss) von 0,1-2,5 mg Scopolamin, das entsprechend dem oben angegebenen Dosisprogramm verabreicht wird, eine therapeutische Wirkung über ungefähr 3 Stunden bis zu 7 Tagen. Daraus folgt auch, dass die konstante Verabreichungsgeschwindigkeit variieren kann, abhängig von dem Körpergewicht (Plasmavolumen) des Patienten. In dieser Beziehung liegt die Geschwindigkeit in den meisten Fällen im Bereich von 1-15 µg/h für Erwachsene und 0,5-10 µg/h für Kinder.

Die Auflage kann aus einem mehrschichtigen Laminat bestehen, das von oben gesehen aus folgenden vier Schichten zusammengesetzt ist:

a) eine schützende Rückseite; b) ein gelartiges Mineralöl-Polyisobuten-Wirkstoff-Reservoir mit dem Wirkstoff, das die Quelle für die konstante Dosierung darstellt; c) eine semipermeable Membran, die die konstante Abgabegeschwindigkeit zum Teil steuert und d) eine gelartige Mineralöl-Polyisobuten-Wirkstoff-Klebeschicht, die als Quelle für die anfängliche Stossdosis dient, sowie aus Klebemitteln, mit denen die Auflage auf der Haut befestigt wird.

Vorzugsweise verwendet man für die Deckschicht a) ein Laminat aus Polymerfolie und einer Metallfolie wie z.B. einer Aluminiumfolie. Polymere, die für diese Schicht angewandt werden können, sind beispielsweise Polyäthylen hoher und geringer Dichte, Polypropylen, Polyvinylchlorid und Polyäthylenterephthalat.

In gelartigem Mineralöl-Polyisobuten-Wirkstoff-Reservoir b) ist die Scopolaminbase teils gelöst und teils ungelöst homogen in dem gelförmigen Gemisch von Mineralöl mit einer Viskosität von ungefähr 10 bis 100 cP bei 25°C und einem Polyisobuten dispergiert. Diese Mineralöl-Polyisobuten-Gemische sind ausgezeichnete Haftmittel und dienen zusätzlich dazu, die pflasterartige Auflage zusammenzuhalten. Das Mineralöl wird beispielsweise als Träger für die Scopolaminbase verwendet, welche eine begrenzte Löslichkeit im Mineralöl (ca. 2 mg/ml) aufweist. Die relativen Mengen in der Reservoirschicht werden so gewählt, dass das Mineralöl im wesentlichen während der ganzen Abgabezeit der pflasterartigen Auflage mit der Base gesättigt ist.

Die nächste Schicht des mehrschichtigen Laminats ist eine semipermeable (mikroporöse) Schicht c), deren Poren mit dem oben beschriebenen Mineralöl gefüllt sind, und die die Geschwindigkeit, mit der die Scopolaminbase an die Haut abgegeben wird, steuert. Der Durchfluss von Scopolamin durch die semipermeable Schicht und der Auflagebereich der Membran müssen so gewählt werden, dass das Scopolamin aus der Reservoirschicht im wesentlichen mit konstanter Geschwindigkeit im Bereich von 0,3 bis 15 µg/h an die Haut abgegeben wird, nachdem das transdermale therapeutische System auf der Haut aufgebracht worden ist. Die semipermeable Membran wird aus polymeren Materialien hergestellt, durch die der Wirkstoff mittels Diffusion hindurchtreten kann. Polymere, die für solche Membranen angewandt werden können, sind beispielsweise Polypropylen, Polycarbonate, Polyvinylchlorid, Celluloseacetat, Cellulosenitrat, Polyacrylnitrat und Organopolysiloxan-Kautschuk.

Die als Haftschicht bezeichnete Schicht d) des Laminates setzt sich im Wesentlichen aus den gleichen Bestandteilen wie die bereits beschriebene Schicht b) zusammen und enthält auch als Wirkstoff die Scopolaminbase, welche für die Stossdosierung bei Beginn der Anwendung des Systems verantwortlich ist. Mit Hilfe der stark haftenden Schicht d) wird die pflasterartige Auflage auf der Haut befestigt, nachdem die abziehbare Schutzschicht unmittelbar vor der Anwendung entfernt worden ist.

Die erfindungsgemäss beschriebenen therapeutischen Systeme, welche das Scopolamin kontinuierlich abgeben, können zur Hemmung der Magensekretion bei akuter Gastritis oder bei Ulcuskrankheiten im Magen oder Dünndarm (Duodenum) verwendet werden, ohne dass es zu unangenehmen parasympathologischen Nebenwirkungen, wie z.B. Mundtrockenheit, Sehstörungen, Photophobie, Pulsbeschleunigung und Harnentleerungsstörungen kommen muss.

Wie bereits erwähnt, kann das transdermal wirkende Arzneimittel auch in Ergänzung von Antacida und/oder $H_2$-Rezeptor-Antagonisten, welche oral oder, wie bereits gesagt, auch parenteral verabreicht werden, appliziert werden. Beispielsweise kann $PGE_2$ als säurehemmende Substanz in einer Dosierung von 0,1 bis 0,3 mg verwendet werden, wobei diese Dosis ein- bis sechsmal verabreicht wird, so dass die tägliche Dosis bei 0,1 bis 9,0 mg liegt.

$H_2$-Rezeptor-Antagonisten werden in Dosen von 25 mg bis 250 mg verwendet. Im allgemeinen werden $H_2$-Rezeptor-Antagonisten ein- bis sechsmal täglich oral verabreicht, so dass die Tagesdosis zwischen 25 mg und 1500 mg liegt.

Antacida werden in Dosen von 25 mg bis 500 mg ein- bis sechsmal oral verabreicht, so dass die Tagesdosis zwischen 25 mg und 3000 mg liegt.

Im Verbindung mit der transdermalen Verwendung von Scopolamin vermindern sich die oben angegebenen Werte bei Patienten, die auf Scopolamin ansprechen.

Die transdermalen Dosierungen von Scopolamin erweisen sich als wirksam in einem Bereich von 0,05 bis 15 µg/h bei gemeinsamer oraler oder auch parenteraler Verwendung mit einer oben angegebenen säurehemmenden Substanz.

Die für die transdermale Anwendung benötigten medizinischen Verbände als Arzneimittel werden wie folgt hergestellt:

a) eine Lösung von 29,2 Teilen hochmolekularem Polyisobuten (Vistanex MML-100, mittleres durch Viskositätsmessung bestimmtes Molekulargewicht 1 200 000), 36,5 Teilen niedermolekularem Polyisobuten (Vistanex® LM-MS durch Viskositätsmessung bestimmtes mittleres Molekulargewicht 35 000), 58,4 Teilen Mineralöl (1 cPa.s (10 cP) bei 25°C), 15,7 Teilen Scopolaminbase und 860,2 Teilen Chloroform wird auf eine ungefähr 65 µm dicke Deckschicht aus einem Aluminiumpolyäthylenterephthalat-Laminat (MEDPAR) aufgegossen, wobei eine Scopolaminbasen-Reservoir-Schicht von ungefähr 50 µm Dicke entsteht. Eine Kombination von Haftmittelschicht und abziehbarer Schicht wird auf ähnliche Weise hergestellt, indem auf eine 200 µm dicke mit Silicon behandelte Aluminium-Polyäthylen-Polyäthylenterephthalat-Folie eine Lösung von 31,8 Teilen des hochmolekularen Polyisobutens, 39,8 Teilen des niedermolekularen Polyisobutens, 63,6 Teilen des Mineralöls, 4,6 Teilen der Scopolaminbase und 860,2 Teilen Chloroform aufgegossen wird. Die entstehende Kontakt-Haftmittel-Schicht ist ungefähr 50 µm dick.

Die, wie oben beschrieben, hergestellte Kombination von Deckschicht und Reservoirschicht wird dann auf eine Seite einer 25 µm dicken mikroporösen Polypropylenmembran (Celgard® 2400), gesättigt mit Mineralöl, und die oben beschriebene Schicht aus Haftmittel und abziehbarer Schutzschicht auf die andere Seite auf der Membran aufgebracht. 1 cm² grosse, runde, scheibenförmige Stücke werden aus dem entstandenen 5-schichtigen Laminat ausgestattet. Jede dieser Auflagen setzt anfänglich 130 bis 150 µm/cm² Scopolamin frei und anschliessend mit im wesentlichen konstanter Geschwindigkeit 3 bis 3,5 µm/cm²/h.

b) Eine Lösung von 22,3 Teilen hochmolekularem Polyisobuten, wie in a), 26 Teilen niedermolekularem Polyisobuten, wie in a), 44,9 Teilen Mineralöl (66 cP bei 25°C), 12,8 Teilen Scopolaminbase, 8,8 Teilen Dimethyllaurylamid und 835,2 Teilen Chloroform wurde auf die in a) beschriebene Deckschicht gegossen, wobei eine Scopolaminbase-Reservoir-Schicht von ungefähr 50 µm Dicke entstand. Eine Kombination aus Haftmittelschicht und abziehbarer Schutzschicht wurde ähnlich hergestellt durch Aufgiessen einer Lösung von 23,5 Teilen des hochmolekularen Isobutens, 29,5 Teilen des niedermolekularen Isobutens, 47,6 Teilen des Mineralöls, 7,8 Teilen Scopolaminbase, 9,0 Teilen Dimethyllaurylamid und 882,6 Teilen Chloroform auf die in a) beschriebene mit Silicon behandelte Polyäthylenterephthalatfolie. Die entstehende Kontaktschicht war ungefähr 50 µm dick.

Die oben beschriebene Deckschicht-Reservoir-Kombination wurde dann auf eine Seite einer 25 µm dicken

mikroporösen Polypropylenmembran (Celgard® 2400), die mit dem Mineralöl gesättigt war, aufgebracht und die oben beschriebene Haftmittel-Schutzschicht-Kombination auf die andere Seite der Membran. Es wurden 4 $cm^2$ grosse, runde, scheibenförmige Stücke aus dem entstandenen 5-schichtigen Laminat ausgestanzt. Jede Auflage ist so ausgebildet, dass sie zu Anfang 125 µg/$cm^2$ Scopolamin abgibt und anschliessend mit im wesentlichen konstanter Geschwindigkeit 2 µg/$cm^2$/h.

c) Medizinische Auflagen werden wie unter a) beschrieben hergestellt, wobei jedoch die abziehbare Haftmittelschicht aus einer 127 µm dicken mit Silikon behandelten Polyäthylenterephthalat-Folie besteht und die Auflagefläche 2,5 $cm^2$ beträgt. In vitro Versuche ergeben, dass diese medizinische Auflage im ersten Stoss innerhalb der ersten 2 Stunden 200 µg und in den nachfolgenden 72 Stunden 10 µg/h abgeben.

Die Erfindung wird durch die folgenden, nicht einschränkenden Beispiele näher erläutert. Unter Teilen, wenn nicht anders angegeben, werden immer Gewichtsteile verstanden. Temperaturen werden in Celsiusgraden angegeben.

Beispiel 1: Junge, gesunde Probanden werden einer körperlichen Untersuchung unterzogen, wobei der körperliche Befund, Hämatologie, Blutchemie und Urinbefund erhoben werden. Der Proband darf eine Woche vor und während der Versuchsdauer keine anderen Medikamente einnehmen, um die Ergebnisse der Untersuchung nicht zu verfälschen. Die gewohnte Lebensweise darf während der Studie nicht geändert und Exzesse jeder Art vermieden werden.

Tabelle I
Wirkung von TTS-Scopolamin auf die basale Magensäuresekretion

| Zeit | HCl (mval/ℓ) $\bar{x} \pm s$ | Test-versus[1] Vorwert |
|---|---|---|
| vor TTS-Scopolamin | 53 ± 13 | |
| am Tage 2 während TTS-Scopolamin | 31 ± 15 | ** |
| 2 Tage nach TTS-Scopolamin | 22 ± 20 | * |

1) verbundener t-Test; n = 8 Probanden
* = p < 0,05
** = p < 0,01

Bei 8 freiwilligen gesunden Versuchspersonen wird über 48 Stunden Scopolaminbase gemäss beschriebenem transdermalem therapeutischem System postaurikulär appliziert.

Vor Beginn der Untersuchung, sowie am 2. und am 4. Tage der laufenden Studie wird die Säuresekretion (HCl und Volumen) basal gemäss Lindenschmid et al., Zschr. Gastroenterologie 17, 820-826 (1979), bestimmt.

Die Magensekretion wird in 10-Minuten-Abständen durch kontinuierliches Absaugen gesammelt. Die sezernierte Menge Salzsäure wird mittels Titration einer 5 ml Probe gegen 0,1-n Natronlauge ermittelt.

Täglich werden von den Probanden Nebenwirkungen auf einer 100 mm Skala eingetragen. Blutdruck, Puls und Pupillenweite werden am Tag 0, 2 und 4 eingetragen.

Anhand der beiliegenden Tabelle I kann gezeigt werden, dass die Säuresekretion bei Anwendung von TTS-Scopolamin erheblich abnimmt.

Beispiel 2: Medizinische Pflaster (Verbände) werden hinter dem Ohr (postaurikulär) 5 erwachsenen, männlichen am Duodenalulcus erkrankten Patienten im Alter von 21 bis 40 Jahren nach dem Entfernen der abziehbaren Haftfolie angebracht. Eine Untersuchung über eine Periode von 48 Stunden unter Verwendung eines Placebo oder von Scopolamin enthaltenden Pflasters in alternierenden Nächten wird eingeleitet.

Am Mittag, vor Beginn der Untersuchung, wird die Hautfläche hinter dem Ohr gereinigt und mit einem Scopolamin oder Placebo enthaltenden Pflaster versehen. Eine Stunde später wird eine Standardmahlzeit eingenommen. 4 Stunden später wird den Patienten eine Magensonde zugeführt und 1 Stunde danach nehmen die Patienten wiederum eine Standardmahlzeit ein. Von diesem Zeitpunkt an bis zum nächsten Morgen wird keine weitere Nahrung, nur Wasser eingenommen.

5 Stunden nach der letzten Mahlzeit wird der Mageninhalt abgesaugt und verworfen. In den nachfolgenden 9 Stunden wird stündlich die Magensekretion mittels einer Saugpumpe gesammelt. 5 ml der jeweiligen stündlich gesammelten Probe wird für pH-Messungen und für die Säurebestimmung mittels Titration mit 0,1-n Natronlauge bis zum pH-Wert 7 (neutral) verwendet. Die Titration wird unter Verwendung eines automatischen Titrators ausgeführt. Die bei den pH-Messungen verwendeten Elektroden werden nach jeder Bestimmung wieder gegen Standardpufferlösungen geeicht.

5

Die Versuchsergebnisse der 2 aufeinanderfolgenden Nächte werden durch die stündlich ermittelte mittlere Ausscheidung an Säure in Millimol/h und die stündlich ermittelte mittlere Wasserstoffionenkonzentration in Millimol/l angegeben. Für die statistische Analyse wird der nach Wilcoxon bezeichnete "Rank-test" verwendet. Es wird dabei festgestellt, dass sich die stündliche mittlere Säureausscheidung von 6, 8 ± 1,8 mmol/h bei Placeboanwendung in der Nacht auf die mittlere stündliche Säureausscheidung von 1,7 ± 0,4 mmol/h bei Verwendung eines Scopolamin enthaltenden Pflasters verringert. Die Ergebnisse können als statistisch signifikant bezeichnet werden (pO 0,001). Die Wasserstoffionenkonzentration wird nicht beeinflusst (79,1 ± 6,4 mmol/l bei Placeboanwendung; 68,2 ± 3,5 mmol/l) bei Verwendung von Scopolamin enthaltenden Pflastern.

4 der Patienten weisen Mundtrockenheit bei Verwendung von Scopolamin nach einer Nacht auf und einer der Patienten leidet unter Mundtrockenheit bereits kurze Zeit nach Applikation des Scopolamin enthaltenden Pflasters. Ueber andere Nebenwirkungen kann nicht berichtet werden.

Beispiel 3: Scopolamin als anticholinergisch wirksame Substanz kann gemäss dieser Erfindung als transdermal wirkendes Arzneimittel in Ergänzung von konventionellen, die Magensäure hemmenden Substanzen, wie z.B. Histamin $H_2$-Rezeptor-Antagonisten, cytoprotektiven Substanzen und traditionellen neutralisierenden Antacida beispielsweise verwendet werden. Medizinische Pflaster (Verbände) gemäss den vorherigen Beschreibungen unter a), b) und c) (auf den Seiten 8, 9 und 10 beschrieben), können in Verbindung mit anderen die Magensäure hemmenden Substanzen verwendet werden, wenn diese Pflaster auf dem Mastodialbereich von unter Ulcuskrankheiten leidenden Patienten aufgebracht werden, welche auch gleichzeitig einer konventionellen Behandlung gemäss unten angegebenem Schema unterzogen werden.

Tabelle II

| Medikament | Dosierung |
| --- | --- |
| Histamin-$H_2$-Rezeptor-Antagonsit | |
| Cimetidin | 1 g täglich bei Mahlzeiten 6 Wochen lang, wobei die Dosis auf 400 mg bei Dauerbehandlung danach gesenkt wird |
| Cytoprotektive Substanzen | |
| PGE$_2$ | 3 mg PGE$_2$ 3 mal täglich bei Mahlzeiten, wobei die Dosis nach einer Woche Behandlung auf 2 mg 3 mal täglich gesenkt wird |
| Antacida | |
| Aluminium- bzw. Magnesiumsalze | 250 mg 3 mal täglich bei Mahlzeiten, gefolgt von 100 mg 3 mal täglich nach 1-wöchiger Verabreichung. |

Beispiel 4: Ein auf dem transdermalen therapeutischen System beruhendes Pflaster (TTS-Verband), ausgelegt auf eine konstante Abgaberate von 4 µg/cm$^2$/h, welches 0,5 mg Scopolamin in konstanter Rate über eine Zeit von 3 Tagen abgibt, wird von den Probanden hinter dem Ohr (postaurikulär) angebracht.

Die nächtliche Säuresekretion wird in zwei aufeinanderfolgenden Nächten an 6 männlichen an Ulcus erkrankten Patienten, welche 48 Stunden unter spezieller Aufsicht stehen, bestimmt. (Das Durchschnittsalter wird mit 31,2 Jahren angegeben). Mittags, vor jedem Versuch, wird die Hautfläche hinter dem Ohr gereinigt und ein mit eimem Placebo (erster Versuch), oder mit Scopolamin versehenen TTS-Pflaster (zweiter Versuch) auf der Haut aufgebracht. Um 17.00 Uhr (d.h. 5 Stunden danach) wird den Probanden eine Magensonde (10 G-Salem-Magensonde) (Argyle Medical) eingeführt. Standardmahlzeiten werden von den Probanden an beiden Tagen um 13.00 und 18.00 eingenommen. Danach wird nur noch die Einnahme von Wasser gestattet. Der Mageninhalt wird um 23.00 Uhr abgesaugt und verworfen und die Magensekretion wird stündlich durch Absaugen bis 8.00 Uhr gesammelt und, wenn notwendig, wird durch manuelle Aspiration nachgeholfen. 5 ml der jeweils stündlich gesammelten Sekretionsproben werden anschliessend benötigt, um den pH-Wert zu messen und für die Titration mit 1-n Natronlauge, um den pH-Wert 7 (neutral) zu erreichen, wobei ein automatischer Tirator (Radiometer, Copenhagen) verwendet wird. Die pH-Elektroden werden nach jeder Bestimmung gegen eine Standardpufferlösung geeicht.

Alle Probanden sind am Morgen über auftretende Nebenwirkungen befragt worden.

Die Ergebnisse werden durch die stündlich ermittelten mittleren Ausscheidungen an Säure in mmol/h und der stündlich ermittelten mittleren Wasserstoffionenkonzentration [H+] in mmol/l angegeben. Für die statistische Analyse wird der nach Wilcoxon bezeichnete "Rank-test" verwendet.

<u>Auswertung der Ergebnisse</u>

Bei einem der sechs ausgewählten Probanden war bei Verwendung eines TTS-Placebo-Pflasters das aufgesaugte Magensekret von technisch unzufriedener Qualität, so dass dieser Teilnehmer vom weiteren Versuch ausgeschlossen worden ist.

Bei den weiteren 5 Probanden wird festgestellt, dass sich die stündliche (23.00-08.00 Uhr) mittlere Säureausscheidung von 6,8 ± 1,8 mmol/h bei Placeboanwendung in der Nacht auf die mittlere stündliche Säureausscheidung von 1,7 ± 0,4 mmol/h bei Verwendung eines Scopolamin enthaltenden Pflasters verringert. Diese 75%ige Verminderung der Magensäuresekretion kann als signifikant bezeichnet werden (p 0.001). Die Wasserstoffionenkonzentration wird nicht beeinflusst (79,1 ± 6,4 mmol/l bei Placeboanwendung; 68,2 ± 3,5 mmol/l bei Verwendung von Scopolamin enthaltendem Pflaster). Es ist bekannt, dass einige Probanden auf $H_2$-Rezeptor-Antagonisten nicht reagieren. In diesem Falle reagierte eine Versuchsperson nicht auf die Verabreichung von Cimetidin. Bei dieser Versuchsperson wurde die Magensäuresekretion über Nacht bei der Verwendung von TTS-Scopolamin um 88% verringert. Es ist zu beachten, dass wohl die Säuresekretion verringert wird, der pH-Wert (Wasserstoffionenkonzentration) jedoch nicht beeinflusst wird, so dass die bakterielle Flora unverändert bleibt.

Der Magensaft zeichnet sich durch die Abwesenheit von Schleim aus, der bei Verwendung von $H_2$-Rezeptor-Antagonisten, z.B. Cimetidin, in Erscheinung tritt.

Die Ergebnisse dieser Studie haben ergeben, dass TTS-Scopolamin bei einer Abgaberate von 5 µg/h (d.h. bei transdermaler Zufuhr von Scopolamin) die nächtliche Säuresekretion bei Patienten, die an einem Duodenalulcus leiden, signifikant verringert wird. Das transdermale therapeutische System (TTS) gibt die anticholinergisch wirksame Substanz wie z.B. Scopolamin, in einem steten Fluss in einer Zeitspanne von 72 Stunden an.

In analoger Weise kann TTS-Scopolamin, alleine oder in Kombination mit einer weiter oben definierten unabhängig verabreichten säurehemmenden Substanz, wie z.B. im Falle eines $H_2$-Rezeptor-Antagonisten, beispielsweise Cimetidin, dafür verwendet werden, um die Rückfallrate geheilter, vorher an Duodenalulcus erkrankter Patienten, zu senken.

**Patentansprüche**

1. Verwendung von Scopolamin als Anticholinergicum zur Herstellung eines transdermal wirkenden Arzneimittels, welches das Scopolamin kontinuierlich in einer Menge von 0,3 bis 15 µg/h abgibt, zur Hemmung der Magensäuresekretion.

2. Verwendung nach Anspruch 1 von Scopolaminbase als Anticholinergicum zur Herstellung eines transdermal wirkenden Arzneimittels, welches das Scopolamin kontinuierlich in einer Menge von 0,3 bis 15 µg/h abgibt, zur Hemmung der Magensäuresekretion.

3. Verwendung nach Ansprüchen 1 und 2 von Scopolamin bzw. Scopolaminbase als Anticholinergicum zur Herstellung eines transdermal wirkenden Arzneimittels, welches das Scopolamin kontinuierlich in einer Menge von 0,3 - 15 µg/h abgibt, zur Hemmung der Magensäuresekretion als Ergänzung von einem Antacidum und/oder $H_2$-Rezeptor-Antagonisten.

4. Verwendung nach Anspruch 1 von Scopolaminbase als Anticholinergicum zur Herstellung eines transdermal wirkenden Arzneimittels, welches das Scopolamin in einer Anfangsstossmenge von 10 bis 200 g/cm² Haut und anschliessend kontinuierlich in einer Menge von 0,3 bis 15µg/h abgibt zur Hemmung der Magensäuresekretion.

5. Verwendung nach Anspruch 4 von Scopolaminbase als Anticholinergicum zur Herstellung eines transdermal wirkenden Arzneimittels, welches das Scopolamin in einer Anfangsstossmenge von 10 bis 200 µm/cm² Haut und anschliessend kontinuierlich in einer Menge von 0,3 bis 15 µg/h abgibt, zur Hemmung als Magensäuresekretion als Ergänzung von einem Antacidum und/oder $H_2$-Rezeptor-Antagonisten.

**Claims**

1. Use of scopolamine as anticholinergic drug for the preparation of a transdermal pharmaceutical composition which releases scopolamine continuously in an amount of 0.3 to 15 µg/h for inhibiting gastric secretion.

2. Use according to claim 1 of scopolamine base as anticholinergic drug for the preparation of a transdermal pharmaceutical composition which releases scopolamine continuously in an amount of 0.3 to 15 µg/h for inhibiting gastric secretion.

3. Use according to either of claims 1 or 2 of scopolamine or scopolamine base for the preparation of a transdermal pharmaceutical composition which releases scopolamine continuously in an amount of 0.3 to 15 µg/h for inhibiting gastric secretion as a supplement to an antacid and/or $H_2$-receptor blockers.

4. Use according to claim 1 of scopolamine base as anticholinergic drug for the preparation of a transdermal pharmaceutical composition which releases scopolamine at an initial pulse rate of 10 to 200 µg/cm$^2$ of skin and thereafter continuously in an amount of 0.3 to 15 µg/h for inhibiting gastric secretion.

5. Use according to claim 4 of scopolamine base as anticholinergic drug for the preparation of a transdermal pharmaceutical composition which releases scopolamine at an initial pulse rate of 10 to 200 µg/cm$^2$ of skin and thereafter continuously in an amount of 0.3 to 15 µg/h for inhibiting gastric secretion as a supplement to an antacid or $H_2$-receptor blockers.

**Revendications**

1. Application de scopolamine comme anticholinergique à la préparation d'un médicament à action transdermique qui délivre la scopolamine de façon continue en une quantité de 0,3 à 15 µg/h.

2. Application selon la revendication 1 de scopolamine base comme anticholinergique à la préparation d'un médicament à action transdermique délivrant la scopolamine de façon continue en une quantité de 0,3 à 15 µg/h afin d'inhiber la sécrétion d'acide gastrique.

3. Application selon les revendications 1 et 2 de scopolamine ou selon les cas de scopolamine base comme anticholinergique à la préparation d'un médicament à action transdermique qui délivre la scopolamine de façon continue en une quantité de 0,3 - 15 µg/h, pour inhiber la sécrétion d'acide gastrique comme complément d'un antacide et/ou d'un antagoniste des récepteurs $H_2$.

4. Application selon la revendication 1 de scopolamine base à la préparation d'un médicament à action transdermique qui délivre la scopolamine en une quantité choc de départ de 10 à 200 g/cm$^2$ de peau puis de façon continue en une quantité de 0,3 à 15 µg/h pour inhiber la sécrétion d'acide gastrique.

5. Application selon la revendication 4 de scopolamine base comme anticholinergique à la préparation d'un médicament à action transdermique qui délivre la scopolamine en une quantité choc de départ de 10 à 200 µm/cm$^2$ de peau puis de façon continue en une quantité de 0,3 à 15 µg/h aux fins d'inhibition de la sécrétion d'acide gastrique comme complément d'un antacide et/ou d'un antagoniste des récepteurs $H_2$.